# EUROPEAN PATENT SPECIFICATION

(11) **EP 3 099 254 B1**
(45) Date of publication and mention of the grant of the patent: **01.12.2021**
(21) Application number: 15703194.9
(22) Date of filing: 26.01.2015
(51) Int. Cl.: A61B 17/34, A61B 17/00

(54) **TROCAR**
TROKAR
TROCART

(30) Priority: 29.01.2014 CN 201410043983
(43) Date of publication of application: 07.12.2016
(73) Proprietor: Ethicon LLC, Guaynabo 00969 (PR)
(72) Inventor: DANNAHER, William D., Cincinnati, Ohio 45236 (US)
(74) Representative: Carpmaels & Ransford LLP
(86) International application number: PCT/US2015/012848
(87) International publication number: WO 2015/116519

(56) References cited:
- WO-A2-01/91653
- US-A- 5 662 615
- US-A- 5 993 471
- US-A1- 2004 260 244
- US-A1- 2009 240 204
- US-A1- 2010 016 799
- US-A1- 2011 028 794
- US-A1- 2011 087 169
- US-A1- 2013 237 902

## Description

### Technical Field

The present invention relates to a trocar, which is applied to puncturing the abdominal wall of a patient to establish a surgical instrument access for minimally invasive operations or surgical procedures.

### Background Art

Trocars are widely employed in laparoscopy, laparoscopic surgery and other minimally invasive operations or surgical procedures. A trocar may establish an access in the abdominal wall of a patient for a laparoscope or other surgical instruments (a guidewire, a catheter, a filter, a stent etc.) to enter the abdominal cavity for an examination or a surgery.

A trocar generally comprises a trocar sleeve, a sealing structure and an obturator. The obturator and the sealing structure of the trocar are normally connected to each other via a connecting portion (i.e. a thread structure or a buckling structure). After the trocar is used, the sealing structure as well as the trocar sleeve will usually be discarded, and the obturator can be disinfected and recycled for reuse in future surgeries or examinations. Known trocars are disclosed in i.a. US5662615 A and US2009/240204 A1.

When being inserted into the abdominal wall or removed from the abdominal wall, the trocar is subject to the force from the palm of a surgeon. In some cases, the trocar is subject to clockwise or anticlockwise torque from the palm of the surgeon. The connecting portion between the trocar sleeve housing and the sealing structure of the trocar is normally a thread structure, thus the trocar can only bear the torque in one direction (i.e. clockwise direction). Therefore, if the trocar encounters torque in an opposite direction (i.e. anticlockwise direction), the trocar sleeve housing and the sealing structure of the trocar may become separated from each other.

Similarly, as the connecting portion between the trocar sleeve housing and the sealing structure of the trocar is the buckling structure, the trocar sleeve housing and the sealing structure of the trocar are also able to be separated from each other due to the clockwise or anticlockwise torque from the palm of the surgeon.

In addition, among existing trocars, the closing and opening of a CO₂ pathway is usually controlled by a switch valve outside the sealing structure. The surgeon is likely to inadvertently touch or open the switch valve when operating the trocar, which is liable to cause leakage of CO₂ from the abdominal cavity through the opened switch valve.

Therefore, such structures of the trocar are expected to be improved for preventing unexpected separation between the trocar sleeve housing and the sealing structure.

### Disclosure of the Present Invention

The purpose of the present invention is to provide a trocar capable of overcoming the shortcomings mentioned above and preventing the separation between the trocar sleeve housing and the sealing structure due to the torque exerted to the joined portion between the trocar sleeve housing and the sealing structure by the surgeon. Furthermore, the trocar provided by the present invention can preferably prevent inadvertent opening of the switch valve of the gas (i.e. CO₂) pathway of the trocar, thus ensuring there is no unintended venting of gas (i.e. CO₂) from the abdominal cavity.

The trocar provided by the present invention is defined by claim 1 and comprises: a trocar sleeve comprising a bowl-shaped proximal end housing part, a distal end portion, and a sheath portion between the two; a sealing structure comprising a top shell having an outer wall and a bottom shell integrally connected with each other, wherein the sealing structure is connected to the proximal end housing portion of the trocar sleeve in a sealed manner and is removable, thus enabling the bottom shell of the sealing structure to be contained in the proximal end housing portion of the trocar sleeve and the outer wall of the top shell of the sealing structure to be exposed proximally to the proximal end housing portion of the trocar sleeve when the trocar is assembled; and an obturator comprising a central shaft and a proximal end portion which is located at the proximal end of the central shaft and adapted to be held by a surgeon, wherein the axial direction of the trocar is defined by the extension direction of the obturator which has an obturator tip at the distal end to perform puncturing, and the central shaft runs through the sheath portion of the trocar sleeve and the sealing structure in a sealed manner and the obturator tip of the central shaft extends out of the distal end of the trocar sleeve and the proximal end portion of the obturator is positioned proximally to the top shell of the sealing structure when the trocar is assembled; wherein the proximal end portion of the obturator, the top shell of the sealing structure and the proximal end housing portion of the trocar sleeve form a head of the trocar when the trocar is assembled, the axial dimensions of the proximal end portion of obturator, the top shell of the sealing structure and the proximal end portion of the trocar sleeve in the axial direction of the puncture device respectively are d1, d2 and d3, and d3 is equal to or greater than 1/2 of the sum of d1, d2 and d3.

Preferably, d3 is greater than 60% of the sum of d1, d2 and d3.

Preferably, d3 is greater than 70% of the sum of d1, d2 and d3.

Preferably, the outer surface of the bottom shell of the sealing structure has an outer thread structure, the inner surface of the proximal end housing portion of the trocar sleeve has inner threaded holes, the outer thread structure of the bottom shell of the sealing structure is joined with the inner threaded holes of the proximal end housing portion of the trocar sleeve when the trocar is assembled, and an elastic sealing element is arranged between the two.

Preferably, the proximal end housing portion of the trocar sleeve has an anti-slipping portion on the peripheral wall thereof.

In all embodiments of the invention, the top shell of the sealing structure is provided with a valve for controlling the opening and closing of gas passage of the trocar; and the valve comprises a valve body, a valve core within the valve body, and an operating handle capable of driving the valve core to rotate within the valve body, wherein the valve body is integral as a unit to the top shell of the sealing structure, the valve core extends in an approximately transverse direction perpendicular to the axial direction of the trocar or approximately in a direction forming an acute angle with the ends of the sealing structure, and the extension direction of the operating handle is approximately perpendicular to that of the valve core.

Preferably, the valve is also provided with a locking device which can be locked so that the operating handle of the valve is not able to drive the valve core to rotate. Preferably, the valve body of the valve is connected to a gas flow tube for supplying gas.

Preferably, the proximal end portion of the obturator is an approximately umbrella-shaped or flat-roofed structure without having a pointy tip; the proximal end portion of the obturator, the top shell of the sealing structure and the proximal end housing portion of the trocar sleeve form the head of the trocar; and the two neighboring parts of the head of the trocar are in smooth transition.

Preferably, a circular passage for gas is formed in the top shell of the sealing structure; the valve body of the valve is located close to the top shell of the sealing structure and integral as a unit to the top shell; and a pathway is formed in the valve body and interconnected to the said circular passage.

Preferably, the proximal end housing portion of the trocar sleeve has a round-roofed outer surface; the sheath portion of the trocar sleeve, which is adjacent to the proximal end housing part, has a columnar outer surface; the round-roofed outer surface and the columnar outer surface are formed of the same material and integral as a unit with each other, forming a trumpet-shaped outer surface, thus enabling the surgeon to operate the trocar by pushing against the proximal end portion of the central shaft by their palm and pressing the trumpet-shaped outer surface by their index finger and middle finger.

The trocar provided by the present invention has a prominent effect in use, separation of the trocar sleeve housing and the sealing structure due to the torque exerted to the joined portion between the trocar sleeve and the sealing structure by the surgeon can be generally prevented. In addition, the trocar provided by the present invention can also prevent unexpected opening of the switch valve of the gas (i.e. CO₂) pathway of the trocar, thus ensuring no leakage of gas (i.e. CO₂) from the abdominal cavity.

With detailed description and relevant drawings below, the other aspects of the present invention, its purposes and advantages of the trocar provided by the present invention will become more easily observable.

### Brief Description of the Drawings

FIG. 1a is a breakdown view of the trocar 1 in Embodiment 1 of the present invention.
FIG. 1b is a stereoscopic view showing the assembled trocar 1 in Embodiment 1 of the present invention.
FIG. 1c is a front view showing the assembled trocar 1 in Embodiment 1 of the present invention.
FIG. 2 is a stereoscopic view showing the assembled sealing structure 200 of the trocar 1 as illustrated in FIG. 1.
FIG. 3a and FIG. 3b are stereoscopic views showing the sealing structure 200 of FIG. 2 observed from different viewpoints.

For different views, corresponding parts are marked with the same symbols, and unnecessary descriptions of the parts are omitted.

### Specific Embodiments

To facilitate the description, "distal end" as cited in the application refers to one end that is far away from the surgeon but close to the abdominal cavity of the patient when the trocar 1 (or component thereof) is in use, and "proximal end" refers to one end that is close to the surgeon when the trocar 1 is in use.

As one example of the present invention, the trocar 1 in Embodiment 1 of the present invention comprises an obturator 100, a sealing structure 200 and a trocar sleeve 300. According to the present invention, the sealing structure 200 may be a disposable component, and the obturator 100 and the trocar sleeve 300 after being used may be disinfected for reuse. The following paragraphs will respectively describe the structures, connection relations and functions of the obturator 100, the sealing structure 200 and the trocar sleeve 300.

### Obturator 100

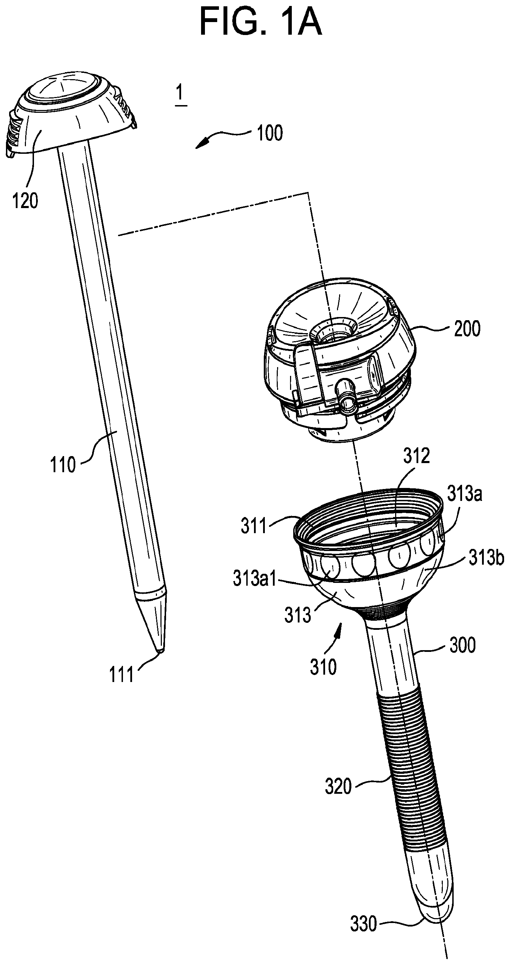

As shown in FIG. 1a, the obturator 100 comprises a central shaft 110 and a proximal end portion 120 which is located at the proximal end of the central shaft 110 and designed to be held by the surgeon. Preferably, the central shaft 110 and the proximal end portion 120 of the obturator 100 are integral as a single structure with each other by injection molding.

According to the present invention, the obturator 100 after being used may be disinfected for reuse in a next surgery, thus the obturator 100 is not a disposable component. When the trocar 1 is assembled, the length extension direction of the central shaft 110 of the obturator 100 in line with the axial direction of the trocar 1, and the radial direction of the trocar 1 is perpendicular to or orthogonal to the axial direction of the trocar 1.

The proximal end portion 120 of the obturator 100 is an approximately umbrella-shaped or flat-roofed structure without having a pointy tip. The outer surface of the proximal end portion 120 may smoothly transit or extend to a top shell 210 of the sealing structure 200. When the trocar 1 is in use, the proximal end portion 120 and the top shell 210 of the sealing structure 200 are against the palm of the surgeon, thus enabling the approximately uniformly distributed force to act upon the palm of the surgeon in a large area.

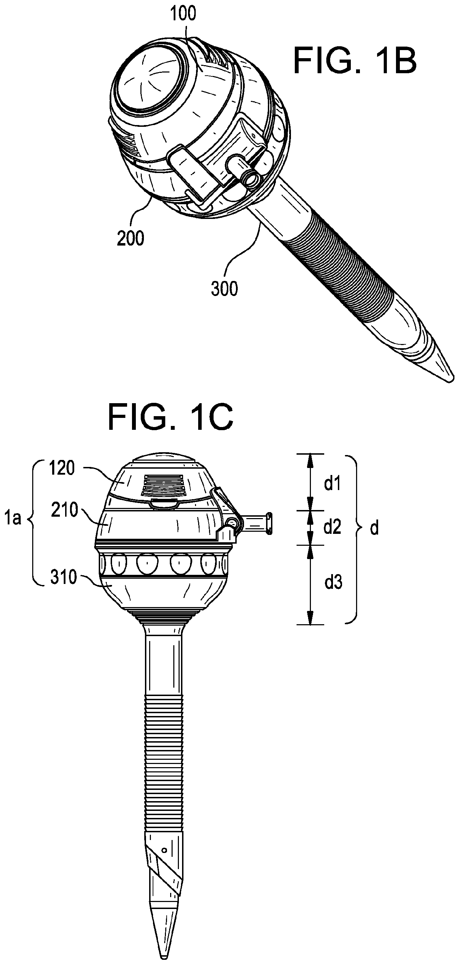

The proximal end portion 120 of the obturator 100 has an axial dimension d1 along the axial direction of the trocar 1, as shown in FIG. 1c. When the trocar 1 is assembled, the proximal end portion 120 of the obturator 100, together with the top shell 210 of the sealing structure 200 and a proximal end housing portion 310 of the trocar sleeve 300, forms the head 1a of the trocar 1. During the process of forming an instrument access for minimally invasive surgeries and operating the instrument, the surgeon may hold the head 1a of the trocar 1 to operate. During the foregoing process, the thrust, tension, clockwise torque or anticlockwise torque may act upon the head 1a of the trocar 1.

The central shaft 110 of the obturator 100 has an elongated slim shape. A blunt obturator tip 111, which is employed to puncture the abdominal wall of the patient to establish an access, is arranged at the distal end of the central shaft 110. Preferably, the obturator tip 111 is shaped with a round head, with the diameter of about 1mm, rather than completely sharp. The obturator tip 111 in such a shape can puncture the abdominal wall of the patient without damaging other tissues of the body.

During assembling the trocar 1, central shaft 110 of the obturator 100 moves towards the distal end in a manner of running through the sealing structure 200 and the trocar sleeve 300 until the bottom of the proximal end portion 120 of the obturator 100 is pressed against the ends at the proximal end of the top shell 210 of the sealing structure 200. The obturator tip 111 of the central shaft 110 extends out of the distal end of the trocar sleeve 300. Thus, the trocar 1 can puncture the abdominal wall of the patient to enter the abdominal cavity in the puncture operation, thereby forming the instrument access for minimally invasive surgeries.

### Sealing Structure 200

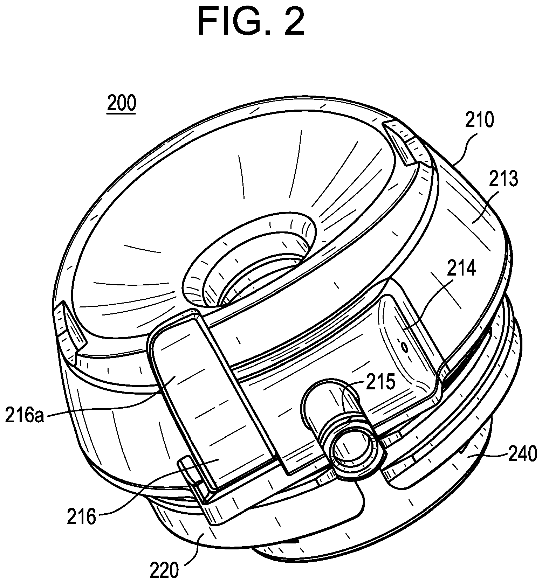

The sealing structure 200 is a disposable component, which will be discarded after use. As shown in FIG. 2, the sealing structure 200 has a top shell 210 and a bottom shell 220. During manufacturing of the sealing structure 200, the top shell 210 and the bottom shell 220 are pressed against each other and then joined by ultrasonic welding to form an integral structure.

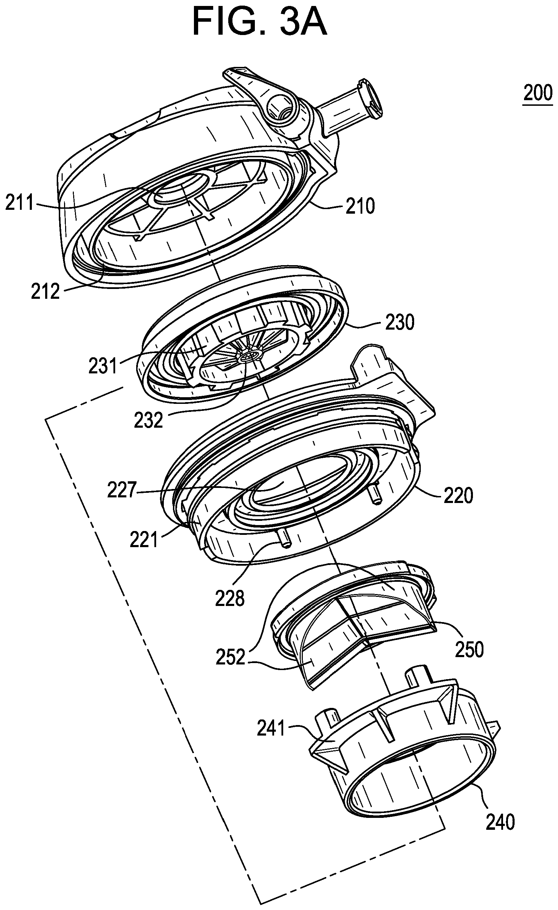

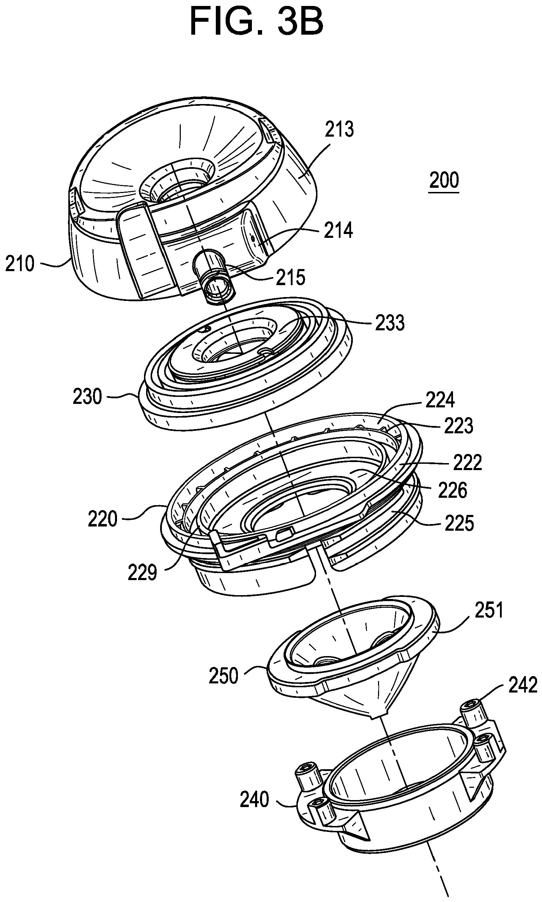

The top shell 210 of the sealing structure 200 approximately resembles a cap or shield. As shown in FIG. 3a and FIG. 3b, a through hole 211 is formed at the center of the top shell 210 for passage of central shaft 110 of the obturator 100.

The top shell 210 has an outer wall 213 and an axial dimension d2 along the axial direction of the trocar 1, as shown in FIG. 1. When the trocar 1 is assembled, the outer wall 213 of the top shell 210 is likely to be exposed outside, forming a middle portion of the head 1a of the trocar 1.

The top shell 210 has an inner peripheral wall 212 integrally formed on the inner side at the proximal end. The inner peripheral wall 212 is roughly perpendicular to the ends at the proximal end of the top shell 210 and extends towards the distal end of the sealing structure 200. The inner peripheral wall is in sealed connection with the inner peripheral flange 229 of the bottom shell 220. The inner peripheral wall 212 of the top shell 210, (the inner peripheral flange 229 of the bottom shell 220) and the inner side of the outer wall 213 of the top shell 210 form a circular space or a circular passage for CO₂.

The outer surface of the outer wall 213 of the top shell 210 extends approximately conically with respect to the axial direction of the trocar 1. A valve body 214 is arranged on the outer wall 213, and a gas flow tube 215 is arranged in the valve body 214. The gas flow tube 215 is interconnected to a CO₂ source (unmarked) to supply CO₂ to the abdominal cavity. Each component connected to the gas flow tube 215 will be described in details in the following paragraphs.

The bottom shell 220 of the sealing structure 200 is approximately cylindrical. The bottom shell 220 has an outer peripheral wall 221, an outer peripheral flange 222 and an inner peripheral flange 229. The outer peripheral flange and the inner peripheral flange are respectively connected to the outer peripheral wall 221 and located on the inner side of the outer peripheral wall 221. The outer peripheral flange 222 and the inner peripheral flange 229 are connected to each other through a plurality of short ribs extending in the radial direction, thus forming a plurality of holes 224 distributed circularly between the outer peripheral flange 222 and the inner peripheral flange 229, so that CO₂ can flow between the gas flow tube 215 and the distal end of the sealing structure 200.

An outer thread structure 225, which is connected to the trocar sleeve 300, is arranged on the outer side of the outer peripheral wall 221 of the bottom shell 220. The bottom shell 220 also has an end wall 226. An opening 227 is formed at the center of the end wall 226 to contain an extended portion 231 of a first sealing element 230 which is arranged between the top shell 210 and the bottom shell 220. The first sealing element 230 approximately resembles a disc, which is provided with an elastic body 233 at the top. The elastic body 233 is formed of rubber. The extended portion 231 having a bottom is formed at the center of the first sealing element 230. A through hole 232 is formed at the center of the bottom of the extended portion 231. The through hole 232 is in tight sealed fit with the central shaft 110 of the obturator 100 penetrating through the through hole 232.

When assembling the sealing structure 200, the top shell 210 and the bottom shell 220 of the sealing structure 200 are subjected to ultrasonic welding to form an integral structure. With the first sealing element 230, a closed passage for CO₂ is formed on the inner peripheral wall 212 of the top shell, the inner peripheral flange 229 of the bottom shell 220 and the outer wall 213 of the top shell. After flowing into the sealing structure 200 through the gas flow tube 215 and the holes 224, CO₂ flows to the trocar sleeve 300 through the said closed passage to enter the abdominal cavity of the patient.

In addition to the top shell 210, the bottom shell 220 and the first sealing element 230, the sealing structure 200 is also provided with a retainer 240 and a second sealing element 250.

As shown in FIG. 3a and FIG. 3b, the overall shape of the retainer 240 resembles an open cylinder. The outer periphery of the retainer 240 has an extended portion 241 in which a plurality of inner threaded holes 242 are formed.

When assembling the sealing structure 200, a plurality of pins 228 as shown in FIG. 3a penetrate through the end wall 226 of the bottom shell 220 and are connected to the inner threaded holes 242 of the extended portion 241 of the retainer 240 by thread. In such a way, the top shell 210, the bottom shell 220 and the retainer 240 are assembled.

In addition, the second sealing element 250 is similarly arranged between the retainer 240 and the bottom shell 220, as shown in FIG. 3a and FIG. 3b. The second sealing element 250 plays as a check valve. According to one preferred embodiment, the second sealing element 250 adopts a duckbill valve, comprising an approximately circular frame 251 and two valve petals 252 formed of an elastic material. Preferably, the elastic material is a material made of flexible neoprene and artificial fibers by special processing.

In absence of ambient pressure, the two valve petals 252 of the second sealing element 250 are kept closed due to elastic action of their own. As central shaft 110 of the obturator 100 is thrust to pass through the space between the two valve petals 252 of the second sealing element 250, the two valve petals 252 are likely to be separated from each other. Therefore, central shaft 110 of the obturator 100 may pass through the space between the two valve petals 252 in a sealed manner, thereby further contacting with the abdominal wall of the patient to form the instrument access for minimally invasive surgeries.

### Trocar Sleeve 300

As shown in FIG. 1a, the trocar sleeve 300 has a proximal end housing portion 310, a slope-cut tip portion 330, and a slim and hollow sheath portion 320 between the two.

The structures and functions of the proximal end housing portion 310 and the hollow sheath portion 320 will be described in the following paragraphs.

The proximal end housing portion 310 is a Luer taper, the diameter or dimension of which is greatly greater than that of the tip portion 330 and the hollow sheath portion 320. As shown in FIG. 1a, the proximal end housing portion 310 approximately resembles a bowl or a round roof, comprising a trumpet-shaped outer surface and smoothly transiting or extending to the hollow sheath portion 320. The proximal end housing portion 310 has an axial dimension d3 along the axial direction of the trocar 1, as shown in FIG. 1c. When the trocar 1 is assembled, the proximal end portion 120 of the obturator 100, the top shell 210 of the sealing structure 200, and the proximal end housing portion 310 of the trocar sleeve 300 form the head 1a of the trocar 1. In addition, the proximal end portion 120 of the obturator 100, the top shell 210 of the sealing structure 200, and the proximal end housing portion 310 of the trocar sleeve 300, are in smooth transition with each other. During the process of forming the instrument access for minimally invasive surgeries and operating the instrument, the surgeon may hold the head 1a of the trocar 1 to exert thrust, tension, clockwise torque or anticlockwise torque to the proximal end housing portion 310 of the trocar sleeve 300.

The proximal end housing portion 310 comprises a peripheral wall 313 and a cavity portion 312 defined by the peripheral wall 313. When the trocar 1 is assembled, the cavity portion 312 of the proximal end housing portion 310 contains the bottom shell 220, the second sealing element 250 and the retainer 240 of the sealing structure 200.

The peripheral wall 313 comprises a sheath portion 313a and an approximately conical portion 313b smoothly transiting from the sheath portion 313a to the distal end.

The proximal end housing portion 310 has inner threaded holes 311 in the inner peripheral wall thereof. During assembling the sealing structure 200, the inner threaded holes 311 of the proximal end housing portion 310 is connected to the outer thread structure 225 of the outer peripheral wall 221 of the bottom shell 220 of the sealing structure 200. In such a way, the bottom shell 220 of the sealing structure 200 can be attached the proximal end housing portion 310 of the trocar sleeve 300 or be detached.

According to the trocar 1 provided by the present invention, the inner threaded holes 311 of the proximal end housing portion 300 of the trocar sleeve 300 is joined with the outer thread structure of the bottom shell 220 of the sealing structure 200 with threads, thus the trocar sleeve 300 after being used can be easily separated from the sealing structure 200 for disinfection for reuse in other surgeries. Therefore, the trocar sleeve 300 is not a disposable component.

To prevent the leakage of CO₂ at the joined portion between the sealing structure 200 and the trocar sleeve 300, an elastic sealing element, such as an elastic sealing gasket, may be arranged at the joined part. The elastic sealing element is tightly squeezed when the inner threaded holes 311 of the trocar sleeve 300 are connected to the outer thread structure 225 of the sealing structure 200.

In addition, the bottom of the cavity portion 312 of the proximal end housing portion 310 is perforated, so that the bottom can be interconnected to an inner axial hole of the hollow sheath portion 320.

The hollow sheath portion 320 of the trocar sleeve 300 has an inner axial hole (unmarked) along the axial direction. The inner diameter of the inner axial hole is equal to or greater than the outer diameter of the central shaft 110 of the obturator 100. The central shaft 110 of the obturator 100 is able to slide to insert into and pass through the inner axial hole of the hollow sheath portion 320 of the trocar sleeve 300, and sealing may be realized between the two. When the proximal end housing portion 310 of the trocar sleeve 300 is joined with the bottom shell 220 of the sealing structure 200 with thread connection, the obturator tip 111 of the central shaft 110 is able to extend out of the tip portion 330 of the trocar sleeve 300.

When the trocar 1 as shown in FIG. 1c is assembled, the overall axial dimension of the head 1a of the trocar 1 is d. As an important improvement of the present invention, the axial dimension d of the head 1a of the trocar 1 is equal to the sum of the axial dimension d1 of the proximal end portion 120 of the obturator 100, the axial dimension d2 of the top shell 210 of the sealing structure 200 and the axial dimension d3 of the proximal end housing portion 310 of the trocar sleeve 300.

According to the present invention, d3 is greater than or equal to 1/2 of d. In other words, the axial dimension of the proximal end housing portion 310 of the trocar sleeve 300 occupies a half portion or a larger portion of the head 1a of the trocar 1.

Furthermore, preferably, d3 is equal to or greater than 60% of d. Even more preferably, d3 is equal to or lager than 70% of d.

According to the present invention, as the surgeon holds the head 1a of the trocar 1 to operate, the point of action is generally at the proximal end housing portion 310 of the trocar sleeve 300. Regardless of the thrust, tension or torque exerted by the surgeon, the force is unlikely to act upon the joined portion between the sealing structure 200 and the trocar sleeve 300 in most cases. Therefore, the thread connection between the outer thread structure 225 of the bottom shell 220 of the sealing structure 200 and the inner threaded holes 311 of the proximal end housing portion 310 of the trocar sleeve 300 is unlikely to be loosened, and unexpected separation between the sealing structure 200 and the trocar sleeve 300 can be prevented. Accordingly, CO₂ in the inner passage of the trocar sleeve 300 is unlikely to leak.

Preferably, the peripheral wall 313 of the proximal end housing portion 310 of the trocar sleeve 300 has an anti-slipping portion 313a1, so that the surgeon can easily hold the trocar sleeve 300 or exert torque onto the trocar sleeve 300.

Preferably, the anti-slipping portion 313a1 refers to a plurality of elliptical recesses uniformly distributed on the peripheral wall 313. The design of the recesses in the peripheral wall 313 is also beneficial to the demolding operation during the molding process of the trocar sleeve 300.

Another important improvement of the present invention involves a valve 216 on the top shell 210 of the sealing structure 200 and the valve body 21 of the valve. As shown in FIG. 2, the valve body 214 extending outwards is integrally formed on the top shell 210 of the sealing structure 200, and the valve 216 is arranged in the valve body 214. The valve 216 is controllably connected to the gas flow tube 215 which is interconnected to the CO₂ source.

The valve body 214 is located close to the outer wall 213 of the top shell 210 and integral as a unit with the outer wall 213 of the top shell 210. In other words, the valve body 214 is directly interconnected to the circular space or the circular passage in the top shell 210 without an intermediate fitting, thus the top shell 210 of the sealing structure 200 and the valve body 214 thereof can be integrally molded by injection molding. A pathway is formed in the valve body 214 of the valve 216, which is interconnected to the circular passage in the top shell 210 and the gas flow tube 215 interconnected to the CO₂ source, thus enabling CO₂ to flow into the abdominal cavity of the patient through the sealing structure 200 and the trocar sleeve 300.

The above-mentioned structures are particularly convenient and beneficial to the molding of the sealing structure 200 of the trocar 1. For a common trocar, the valve 216 is normally in a crossed structure, and the valve 216 and the sealing structure 200 are two separated components, thus the valve 216 and the sealing structure 200 are required to be welded together before assembling the trocar 1. Unlike common trocar, the trocar 1 provided by the present invention is advantageous in that there is no need for pipe to connect the valve 216 to the top shell 210 of the sealing structure 200, thus saving materials used for these connecting pipes.

In addition, in prior molding processes, three inserts are required. However, the trocar 1 provided by the present invention is advantageous in that the molding process of the sealing structure 200 only requires two inserts perpendicular to each other. In comparison with the prior art, the molding process of the sealing structure 200 of the trocar 1 provided by the present invention is simpler. The valve body 214 of the sealing structure 200 and the valve 216 are specifically shown in FIG. 2. The valve 216 comprises the valve body 214, a valve core (unmarked) in the valve body 214, and an operating handle 216a. The valve core is arranged in the valve body 214 and can rotate. The valve core of the valve 216 extends along a transverse direction approximately perpendicular to the axial direction of the trocar 1 and is located in the valve body 214 of the upper valve 210 of the sealing structure 200. The extension direction of the operating handle 216a of the valve 216 is approximately perpendicular to that of the valve core of the valve 216. The surgeon may operate the operating handle 216a of the valve 216 along a direction from the proximal end of the sealing structure 200 to the distal end of the sealing structure 200 (i.e. from top to bottom) or along an opposite direction (i.e. from bottom to top) to drive the valve core to rotate in the valve body 214, thus controlling the opening and closing of the valve 216.

When the surgeon exerts the torque to the operating handle 216a of the valve 216 from top to bottom or from bottom to top, the operating handle 216a of the valve 216 can drive the valve 216 to rotate, thus keeping the valve core of the valve 216 in an open state, a partially open state or a closed state. Correspondingly, the gas flow tube 215 may be connected to, partially connected to or shut from the CO₂ source.

According to the present invention, the valve core of the valve 216 is transversely arranged in the valve body 214 of the top shell 210 of the sealing structure 200. Correspondingly, when the trocar 1 is employed to establish the access in the abdominal wall of the patient or conducting laparoscopy or laparoscopic surgery, the operating handle 216a can only rotate when the surgeon exerts the force or torque to the operating handle 216a in a vertical plane (i.e. from top to bottom or from bottom to top), thus driving the valve core to rotate in order to open (or close) the gas flow tube 215 for CO₂.

Regardless of being at the proximal end of the sealing structure 200 or the distal end of the sealing structure 200, the operating handle 216a of the valve 216 is kept in a contracted state (i.e. not extended), thus the possibility that the gas flow tube 215 connected to the valve 216 hooks unto other articles is reduced. Therefore, the valve 216 and the gas flow tube 215 are unlikely to be opened even the surgeon inadvertently touches the operating handle 216a along a horizontal or transverse direction.

Optionally, the valve core of the valve 216 can be obliquely arranged in the valve body 214 of the top shell 210 in a manner of forming an acute angle with the sealing structure 200. Similarly, the extension direction of the operating handle 216a of the valve 216 is approximately perpendicular to that of the valve core of the valve 216.

Provided that the valve core of the valve 216 is transversely arranged or obliquely arranged, a locking device (unmarked) may be arranged on the valve 216. After the valve 216 is opened or closed, the locking device may be locked, so that the operating handle 216a of the valve 216 cannot drive the valve core to rotate.

Preferably, as shown in FIG. 1a, FIG. 1b and FIG. 1c, the proximal end housing portion 310 of the trocar sleeve 300 has a round-roofed outer surface; and the sheath portion 320 of the trocar sleeve 300, which is adjacent to the proximal end housing portion 310, has a columnar outer surface; the round-roofed outer surface and the columnar outer surface are formed of the same material and integral as a unit with each other, forming a trumpet-shaped outer surface, thus enabling the surgeon to operate the trocar 1 by pushing against the proximal end portion 120 of the obturator 100 by their palm and pressing the trumpet-shaped outer surface by their index finger and middle finger.

For the above-mentioned structures, the surgeon only needs to squeeze the proximal end portion 120 of the obturator 100 and the trumpet-shaped outer surface of the trocar sleeve 300 while operating with the trocar 1, thus avoiding the torque which may separate the sealing structure 200 from the proximal end housing portion 310 of the trocar sleeve 300.

The foregoing paragraphs have described a series of embodiments of the present invention. However, the present invention is not restricted by the above-mentioned descriptions and embodiments illustrated by the drawings. The protection scope of the present invention is defined by the claims attached.

## Claims

1. A trocar (1), comprising:
a trocar sleeve (300) comprising a bowl-shaped proximal end housing part (310), a distal end portion (330), and a sheath portion (320) between the two;
a sealing structure (200) comprising a top shell (210) having an outer wall (213) and a bottom shell (220) integral with each other, wherein the said sealing structure (200) can be connected to the proximal end housing portion (310) of the trocar sleeve (300) in a sealed manner and is removable, thus enabling the bottom shell (220) of the sealing structure (200) to be contained in the proximal end housing portion (310) of the trocar sleeve (300) and the outer wall (213) of the top shell (210) of the sealing structure (200) to be exposed proximally to the proximal end housing portion (310) of the trocar sleeve (300) when the trocar (1) is assembled; and
an obturator (100) comprising a central shaft (110) and a proximal end portion (120) which is located at the proximal end of the central shaft (110) and adapted to be held by a surgeon, wherein the axial direction of the trocar (1) is defined by the extension direction of the central shaft (110) which also has a obturator tip (111) at the distal end to perform puncturing, wherein the central shaft (110) runs through the sheath portion (320) of the trocar sleeve (300) and the sealing structure (200) in a sealed manner, the obturator tip (111) of the central shaft (110) extends out of the distal end portion (330) of the trocar sleeve (300) and the proximal end portion (120) of the obturator (100) is positioned proximally to the top shell (210) of the sealing structure (200) when the trocar (1) is assembled;
wherein the proximal end portion (120) of the obturator (100), the top shell (210) of the sealing structure (200) and the proximal end housing portion (310) of the trocar sleeve (300) form a head (1a) of the trocar (1) when the trocar (1) is assembled, the axial dimensions of the proximal end portion (120) of the obturator (100), the top shell (210) of the sealing structure (200) and the proximal end housing portion (310) of the trocar sleeve (300) in the axial direction of the trocar (1) respectively are d1, d2 and d3, and d3 is equal to or greater than 1/2 of the sum of d1, d2 and d3; and
**characterized in that** the top shell (210) of the sealing structure (200) is provided with a valve (216) for controlling the opening and closing of a gas passage for the trocar (1); the valve (216) comprising a valve body (214), a valve core within the valve body (214), and an operating handle (216a) capable of driving the valve core to rotate in the valve body (214), wherein the valve body (214) is integral with the top shell (210) of the sealing structure (200), the valve core extends in an approximately transverse direction perpendicular to the axial direction of the trocar (1) or approximately in a direction forming an acute angle with the ends of the sealing structure (200), and the extension direction of the operating handle (216a) is approximately perpendicular to that of the valve core.

2. The trocar (1) according to Claim 1, **characterized in that** d3 is greater than 60% of the sum of d1, d2 and d3.

3. The trocar (1) according to Claim 1 or 2, **characterized in that** d3 is greater than 70% of the sum of d1, d2 and d3.

4. The trocar (1) according to any preceding Claim, **characterized in that** the outer surface of the bottom shell (220) of the sealing structure (200) has an outer thread structure (225), the inner surface of the proximal end housing portion (310) of the trocar sleeve (300) has inner threaded holes (311), the outer thread structure (225) of the bottom shell (220) of the sealing structure (200) is joined with the inner threaded holes (311) of the proximal end housing portion (310) of the trocar sleeve 9300) when the trocar (1) is assembled, and an elastic sealing element is arranged between the two.

5. The trocar (1) according to any preceding Claim, **characterized in that** the proximal end housing portion (310) of the trocar sleeve (300) has an anti-slipping structure (313a1) on the peripheral wall (313) thereof.

6. The trocar (1) according to any preceding Claim, **characterized in that** the valve (216) is also provided with a locking device which can be locked so that the operating handle (216a) of the valve (216) is not able drive the valve core to rotate.

7. The trocar (1) according to any preceding Claim, **characterized in that** the valve body (214) of the valve (216) is connected to a gas flow tube (215) for supplying gas or venting.

8. The trocar (1) according to any preceding Claim, **characterized in that** the proximal end portion (120) of the obturator (100) is an approximately umbrella-shaped or flat-roofed structure without having a pointy tip; the proximal end portion (120) of the obturator (100), the top shell (210) of the sealing structure (200) and the proximal end housing portion (310) of the trocar sleeve (300) form the head (1a) of the trocar (1); and the two neighboring parts forming the head (1a) of the trocar (1) are in smooth transition.

9. The trocar (1) according to any preceding Claim, **characterized in that** a circular passage for gas is formed in the top shell (210) of the sealing structure (200); the valve body (214) of the valve (216) is located close to the top shell (210) of the sealing structure (200) and integral with the top shell (210); and a pathway is formed in the valve body (214) and interconnected to the said circular passage.

10. The trocar (1) according to any preceding Claim, **characterized in that** the proximal end housing portion (310) of the trocar sleeve (300) has a round-roofed outer surface; the sheath portion (320) of the trocar sleeve (300), which is adjacent to the proximal end housing part (310), has a columnar outer surface; the round-roofed outer surface and the columnar outer surface are formed of the same material and integral with each other, forming a trumpet-shaped outer surface, thus enabling the surgeon to operate by pushing against the proximal end portion (120) of the obturator (100) by their palm and pressing the trumpet-shaped outer surface by their index finger and middle finger.

## Patentansprüche

1. Trokar (1), umfassend:
eine Trokarhülse (300), umfassend einen schüsselförmigen proximalen Endgehäuseteil (310), einen distalen Endabschnitt (330) und einen Hüllenabschnitt (320) zwischen den beiden,
eine Dichtstruktur (200), umfassend eine obere Schale (210) mit einer äußeren Wand (213) und eine untere Schale (220), die miteinander integral sind, wobei die Dichtstruktur (200) dichtend mit dem proximalen Endgehäuseabschnitt (310) der Trokarhülse (300) verbunden werden kann und entfernbar ist, wodurch die untere Schale (220) der Dichtstruktur (200) in dem proximalen Endgehäuseabschnitt (310) der Trokarhülse (300) enthalten sein kann und die äußere Wand (213) der oberen Schale (210) der Dichtstruktur (200) proximal zu dem proximalen Endgehäuseabschnitt (310) der Trokarhülse (300) freiliegen kann, wenn der Trokar (1) zusammengebaut ist, und
einen Obturator (100), umfassend einen mittleren Schaft (110) und einen proximalen Endabschnitt (120), der an dem proximalen Ende des mittleren Schafts (110) angeordnet und dazu ausgeführt ist, von einem Chirurg gehalten zu werden, wobei die axiale Richtung des Trokars (1) von der Erstreckungsrichtung des mittleren Schafts (110) definiert wird, der auch eine Obturatorspitze (111) an dem distalen Ende hat, um den Einstich durchzuführen, wobei der mittlere Schaft (110) abgedichtet durch den Hüllenabschnitt (320) der Trokarhülse (300) und die Dichtstruktur (200) läuft, sich die Obturatorspitze (111) des mittleren Schafts (110) aus dem distalen Endabschnitt (330) der Trokarhülse (300) heraus erstreckt und der proximale Endabschnitt (120) des Obturators (100) proximal zu der oberen Schale (210) der Dichtstruktur (200) positioniert ist, wenn der Trokar (1) zusammengebaut ist,
wobei der proximale Endabschnitt (120) des Obturators (100), die obere Schale (210) der Dichtstruktur (200) und der proximale Endgehäuseabschnitt (310) der Trokarhülse (300) einen Kopf (1a) des Trokars (1) bilden, wenn der Trokar (1) zusammengebaut ist, die axialen Abmessungen des proximalen Endabschnitts (120) des Obturators (100), der oberen Schale (210) der Dichtstruktur (200) und des proximalen Endgehäuseabschnitts (310) der Trokarhülse (300) in der axialen Richtung des Trokars (1) d1, d2 bzw. d3 sind und d3 gleich oder größer als ½ der Summe von d1, d2 und d3 ist, und
**dadurch gekennzeichnet, dass** die obere Schale (210) der Dichtstruktur (200) mit einem Ventil (216) zum Steuern des Öffnens und Schließens eines Gasdurchgangs für den Trokar (1) versehen ist, wobei das Ventil (216) einen Ventilkörper (214), einen Ventilkern in dem Ventilkörper (214) und einen Betätigungsgriff (216a) umfasst, der den Ventilkern zum Drehen in dem Ventilkörper (214) antreiben kann, wobei der Ventilkörper (214) mit der oberen Schale (210) der Dichtstruktur (200) integral ist, sich der Ventilkern in eine senkrecht zu der axialen Richtung des Trokars (1) verlaufende Querrichtung oder ungefähr in eine Richtung erstreckt, die einen spitzen Winkel mit den Enden der Dichtstruktur (200) bildet, und die Erstreckungsrichtung des Betätigungsgriffs (216a) ungefähr senkrecht zu der des Ventilkerns verläuft.

2. Trokar (1) nach Anspruch 1, **dadurch gekennzeichnet, dass** d3 größer als 60% der Summe von d1, d2 und d3 ist.

3. Trokar (1) nach Anspruch 1 oder 2, **dadurch gekennzeichnet, dass** d3 größer als 70% der Summe von d1, d2 und d3 ist.

4. Trokar (1) nach einem der vorhergehenden Ansprüche, **dadurch gekennzeichnet, dass** die Außenfläche der unteren Schale (220) der Dichtstruktur (200) eine Außengewindestruktur (225) hat, die Innenfläche des proximalen Endgehäuseabschnitts (310) der Trokarhülse (300) Löcher (311) mit Innengewinde hat, die Außengewindestruktur (225) der unteren Schale (220) der Dichtstruktur (200) mit den Löchern (311) mit Innengewinde des proximalen Endgehäuseabschnitts (310) der Trokarhülse 9300) verbunden ist, wenn der Trokar (1) zusammengebaut ist, und ein elastisches Dichtelement zwischen den beiden angeordnet ist.

5. Trokar (1) nach einem der vorhergehenden Ansprüche, **dadurch gekennzeichnet, dass** der proximale Endgehäuseabschnitt (310) der Trokarhülse (300) eine Antirutschstruktur (313a1) an der Umfangswand (313) davon hat.

6. Trokar (1) nach einem der vorhergehenden Ansprüche, **dadurch gekennzeichnet, dass** das Ventil (216) auch mit einer Verriegelungsvorrichtung versehen ist, die verriegelt werden kann, so dass der Betätigungsgriff (216a) des Ventils (216) nicht in der Lage ist, den Ventilkern zum Drehen anzutreiben.

7. Trokar (1) nach einem der vorhergehenden Ansprüche, **dadurch gekennzeichnet, dass** der Ventilkörper (214) des Ventils (216) mit einem Gasströmungsrohr (215) zur Zufuhr von Gas oder zur Entlüftung verbunden ist.

8. Trokar (1) nach einem der vorhergehenden Ansprüche, **dadurch gekennzeichnet, dass** der proximale Endabschnitt (120) des Obturators (100) eine ungefähr schirmförmige Struktur oder eine Flachdachstruktur ohne scharfe Spitze ist, der proximale Endabschnitt (120) des Obturators (100), die obere Schale (210) der Dichtstruktur (200) und der proximale Endgehäuseabschnitt (310) der Trokarhülse (300) den Kopf (1a) des Trokars (1) bilden und die beiden den Kopf (1a) des Trokars (1) bildenden benachbarten Teile glatt ineinander übergehen.

9. Trokar (1) nach einem der vorhergehenden Ansprüche, **dadurch gekennzeichnet, dass** ein kreisförmiger Durchgang für Gas in der oberen Schale (210) der Dichtstruktur (200) ausgebildet ist, der Ventilkörper (214) des Ventils (216) nahe der oberen Schale (210) der Dichtstruktur (200) angeordnet und integral mit der oberen Schale (210) ist und ein Durchgang in dem Ventilkörper (214) ausgebildet und mit dem kreisförmigen Durchgang verbunden ist.

10. Trokar (1) nach einem der vorhergehenden Ansprüche, **dadurch gekennzeichnet, dass** der proximale Endgehäuseabschnitt (310) der Trokarhülse (300) eine Außenfläche mit rundem Dach hat, der Hüllenabschnitt (320) der Trokarhülse (300), der dem proximalen Endgehäuseteil (310) benachbart ist, eine säulenförmige Außenfläche hat und die Außenfläche mit rundem Dach und die säulenförmige Außenfläche aus demselben Material ausgebildet und miteinander integral sind und eine trompetenförmige Außenfläche bilden, wodurch der Chirurg operieren kann, indem er mit der Handfläche gegen den proximalen Endabschnitt (120) des Obturators (100) drückt und den Zeigefinger und den Mittelfinger gegen die trompetenförmige Außenfläche presst.

## Revendications

1. Trocart (1), comprenant :
une chemise de trocart (300) comprenant une partie logement d'extrémité proximale en forme d'écuelle (310), une partie d'extrémité distale (330) et une partie gaine (320) entre les deux ;
une structure d'étanchéité (200) comprenant une coque supérieure (210) comportant une paroi extérieure (213) et une coque inférieure (220) formant un ensemble d'un seul tenant, ladite structure d'étanchéité (200) pouvant être raccordée à la partie logement d'extrémité proximale (310) de la chemise de trocart (300) de manière étanche et étant amovible, de sorte que la coque inférieure (220) de la structure d'étanchéité (200) puisse être logée dans la partie logement d'extrémité proximale (310) de la chemise de trocart (300) et que la paroi extérieure (213) de la coque supérieure (210) de la structure d'étanchéité (200) puisse être exposée en position proximale par rapport à la partie logement d'extrémité proximale (310) de la chemise de trocart (300) lorsque le trocart (1) est assemblé ; et
un obturateur (100) comprenant une tige centrale (110) et une partie d'extrémité proximale (120) qui est située à l'extrémité proximale de la tige centrale (110) et appropriée pour être tenue par un(e) chirurgien(ne), la direction axiale du trocart (1) étant définie par la direction d'étendue de la tige centrale (110) qui comporte également une partie terminale d'obturateur (111) au niveau de l'extrémité distale pour réaliser une perforation, la tige centrale (110) s'étendant à travers la partie gaine (320) de la chemise de trocart (300) et la structure d'étanchéité (200) de manière étanche, la partie terminale d'obturateur (111) de la tige centrale (110) s'étendant hors de la partie d'extrémité distale (330) de la chemise de trocart (300) et la partie d'extrémité proximale (120) de l'obturateur (100) étant placée en position proximale par rapport à la coque supérieure (210) de la structure d'étanchéité (200) lorsque le trocart (1) est assemblé ;
la partie d'extrémité proximale (120) de l'obturateur (100), la coque supérieure (210) de la structure d'étanchéité (200) et la partie logement d'extrémité proximale (310) de la chemise de trocart (300) formant une tête (1a) du trocart (1) lorsque le trocart (1) est assemblé, les dimensions axiales de la partie d'extrémité proximale (120) de l'obturateur (100), de la coque supérieure (210) de la structure d'étanchéité (200) et de la partie logement d'extrémité proximale (310) de la chemise de trocart (300) dans la direction axiale du trocart (1) étant respectivement d1, d2 et d3, et d3 étant égal ou supérieur à la moitié de la somme de d1, d2 et d3 ; et
**caractérisé en ce que** la coque supérieure (210) de la structure d'étanchéité (200) est pourvue d'une vanne (216) pour commander l'ouverture et la fermeture d'un passage de gaz pour le trocart (1) ; la vanne (216) comprenant un corps de vanne (214), une pièce intérieure de vanne à l'intérieur du corps de vanne (214), et un levier d'actionnement (216a) apte à entraîner une rotation de la pièce intérieure de vanne dans le corps de vanne (214), le corps de vanne (214) étant réalisé d'un seul tenant avec la coque supérieure (210) de la structure d'étanchéité (200), la pièce intérieure de vanne s'étendant dans une direction approximativement transversale perpendiculaire à la direction axiale du trocart (1) ou approximativement dans une direction formant un angle aigu avec les extrémités de la structure d'étanchéité (200), et la direction d'étendue du levier d'actionnement (216a) étant approximativement perpendiculaire à celle de la pièce intérieure de vanne.

2. Trocart (1) selon la revendication 1, **caractérisé en ce que** d3 est supérieur à 60 % de la somme de d1, d2 et d3.

3. Trocart (1) selon la revendication 1 ou 2, **caractérisé en ce que** d3 est supérieur à 70 % de la somme de d1, d2 et d3.

4. Trocart (1) selon l'une quelconque des revendications précédentes, **caractérisé en ce que** la surface extérieure de la coque inférieure (220) de la structure d'étanchéité (200) comporte une structure filetée extérieure (225), la surface intérieure de la partie logement d'extrémité proximale (310) de la chemise de trocart (300) comporte des trous taraudés intérieurs (311), la structure filetée extérieure (225) de la coque inférieure (220) de la structure d'étanchéité (200) est accouplée avec les trous taraudés intérieurs (311) de la partie logement d'extrémité proximale (310) de la chemise de trocart 9300) lorsque le trocart (1) est assemblé, et un élément d'étanchéité élastique est disposé entre les deux.

5. Trocart (1) selon l'une quelconque des revendications précédentes, **caractérisé en ce que** la partie logement d'extrémité proximale (310) de la chemise de trocart (300) comporte une structure antiglissante (313a1) sur sa paroi périphérique (313).

6. Trocart (1) selon l'une quelconque des revendications précédentes, **caractérisé en ce que** la vanne (216) est également pourvue d'un dispositif de verrouillage qui peut être verrouillé de telle sorte que le levier d'actionnement (216a) de la vanne (216) ne puisse pas entraîner une rotation de la pièce intérieure de vanne.

7. Trocart (1) selon l'une quelconque des revendications précédentes, **caractérisé en ce que** le corps de vanne (214) de la vanne (216) est raccordé à un tube d'écoulement de gaz (215) à des fins d'alimentation en gaz ou d'évacuation.

8. Trocart (1) selon l'une quelconque des revendications précédentes, **caractérisé en ce que** la partie d'extrémité proximale (120) de l'obturateur (100) est une structure approximativement en forme d'ombrelle ou à sommet plat, ne comportant pas de partie terminale pointue ; la partie d'extrémité proximale (120) de l'obturateur (100), la coque supérieure (210) de la structure d'étanchéité (200) et la partie logement d'extrémité proximale (310) de la chemise de trocart (300) forment la tête (1a) du trocart (1) ; et une transition lisse existe entre les deux parties adjacentes formant la tête (1a) du trocart (1).

9. Trocart (1) selon l'une quelconque des revendications précédentes, **caractérisé en ce qu'**un passage circulaire pour du gaz est formé dans la coque supérieure (210) de la structure d'étanchéité (200) ; le corps de vanne (214) de la vanne (216) est situé à proximité de la coque supérieure (210) de la structure d'étanchéité (200) et réalisé d'un seul tenant avec la coque supérieure (210) ; et une voie est formée dans le corps de vanne (214) et reliée audit passage circulaire.

10. Trocart (1) selon l'une quelconque des revendications précédentes, **caractérisé en ce que** la partie logement d'extrémité proximale (310) de la chemise de trocart (300) comporte une surface extérieure à sommet arrondi ; la partie gaine (320) de la chemise de trocart (300), qui est adjacente à la partie logement d'extrémité proximale (310), comporte une surface extérieure colonnaire ; la surface extérieure à sommet arrondi et la surface extérieure colonnaire sont faites du même matériau et constituent un ensemble d'un seul tenant, formant une surface extérieure en forme de trompette, de sorte que le/la chirurgien(ne) puisse effectuer une manipulation en poussant contre la partie d'extrémité proximale (120) de l'obturateur (100) avec sa paume et en appuyant sur la surface extérieure en forme de trompette avec son index et son majeur.
